(19) Europäisches Patentamt
European Patent Office
Office européen des brevets

(11) **EP 3 175 788 B1**

## (12) EUROPEAN PATENT SPECIFICATION

(45) Date of publication and mention
of the grant of the patent:
**06.11.2019 Bulletin 2019/45**

(51) Int Cl.:
*A61B 6/00* *(2006.01)*   *A61B 8/00* *(2006.01)*
*G01R 33/54* *(2006.01)*

(21) Application number: **16173458.7**

(22) Date of filing: **08.06.2016**

(54) **MEDICAL IMAGING APPARATUS AND CONTROL METHOD THEREOF**

VORRICHTUNG ZUR MEDIZINISCHEN BILDGEBUNG UND STEUERUNGSVERFAHREN DAFÜR

APPAREIL D'IMAGERIE MÉDICALE ET SON PROCÉDÉ DE COMMANDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.12.2015 KR 20150169900**

(43) Date of publication of application:
**07.06.2017 Bulletin 2017/23**

(73) Proprietor: **Samsung Electronics Co., Ltd.
Gyeonggi-do 16677 (KR)**

(72) Inventors:
• **KIM, Joo Young
  Gyeonggi-do (KR)**
• **OH, Keum Yong
  Gyeonggi-do (KR)**

(74) Representative: **D'Halleweyn, Nele Veerle Trees
Gertrudis et al
Arnold & Siedsma
Bezuidenhoutseweg 57
2594 AC The Hague (NL)**

(56) References cited:
**EP-A1- 2 713 177      DE-A1-102014 211 686
US-A1- 2014 098 932   US-A1- 2015 281 564**

**Description**

BACKGROUND

**1. Field**

Apparatuses and methods consistent with exemplary embodiments relate to a medical imaging apparatus for displaying a protocol list and a control method thereof.

**2. Description of the Related Art**

In general, medical imaging devices acquire information of a patient and provide an image. The medical imaging devices include an X-ray imaging device, an ultrasonic diagnostic device, a computed tomography device, a magnetic resonance imaging device, etc.

Among these devices, the magnetic resonance imaging device has relatively free imaging conditions and provides excellent contrast in soft tissues and various diagnostic information images, thus occupying a prominent place in the field of diagnosis using medical images. Magnetic resonance imaging (MRI) images the density and physiochemical characteristics of atomic nuclei by generating nuclear magnetic resonance of hydrogen atoms in a human body using a magnetic field that is not harmful to the human body, and radio waves that are a non-ionizing form of radiation.

In more detail, the magnetic resonance imaging device images the inside of a target object by supplying a designated frequency and energy to atomic nuclei in a state in which a designated magnetic field is applied to the inside of a gantry to convert energy discharged from the atomic nuclei into a signal.

The magnetic resonance imaging device has various image parameters applied thereto to generate a magnetic resonance image with respect to an object, and a user may acquire a magnetic resonance image by selecting a set of one or more desired image parameters, that is, a protocol, depending on the situation.

EP 2713177 relates to a medical imaging apparatus operates to automatically recommend protocols suitable for image capture of a subject based on information related to the subject, and a control method operates the medical imaging apparatus.

SUMMARY

Exemplary embodiments may address at least the above problems and/or disadvantages and other disadvantages not described above. Also, the exemplary embodiments are not required to overcome the disadvantages described above, and may not overcome any of the problems described above.

One or more exemplary embodiments provide a medical imaging apparatus capable of grouping one or more protocols that are selectable by a user and intuitively displaying the grouped protocol, and a control method thereof.

One or more exemplary embodiments provide a medical imaging apparatus capable of providing a protocol list in consideration of connection between one or more protocols, and a control method thereof.

According to an aspect of an exemplary embodiment, there is provided a medical imaging apparatus including a display configured to display a list of protocols, each of the protocols having one or more parameters for acquiring a medical image. The medical imaging apparatus further includes an interface configured to receive an input of a parameter for acquiring the medical image, and a controller configured to group the protocols based on the input parameter, and control the display to display the list of the grouped protocols.

The displayed list of the grouped protocols may have a tree structure.

The display may be further configured to display, in the displayed list of the protocols, the one or more parameters of each of the protocols with the respective protocols.

The one or more parameters of each of the protocols may include at least one among a contrast, a resolution, a geometry, a sequence type, slice information, a photography direction and an object referred to, and the input parameter may include one among the contrast, the resolution, the geometry, the sequence type, the slice information, the photography direction and the object referred to.

A first protocol among the protocols may be grouped in a first column of the displayed list of the grouped protocols, and a second protocol among the protocols may be grouped in a second column of the displayed list of the grouped protocols, the second protocol referring to the first protocol. The interface may be further configured to receive an instruction to change a value of a parameter of a protocol among the protocols, and the controller may be further configured to change the value of the parameter of the protocol based on the instruction.

The interface may be further configured to receive a selection of a protocol from the protocols, and the controller may be further configured to generate a pulse sequence and perform an image processing, based on the selected protocol.

The controller may be further configured to control the display to display values of the input parameter in a first column of the displayed list of the grouped protocols, and each of the protocols may be grouped to correspond to a respective one among the values, in a second column of the displayed list of the grouped protocols.

The interface may be further configured to receive an instruction to change a value among the values, and the controller may be further configured to change the value for a protocol grouped to correspond to the value, based on the instruction.

The controller may be further configured to change a name of a protocol among the protocols to have the input parameter, and control the display to display the changed name.

The display may be further configured to display, in the displayed list of the grouped protocols, the one or more parameters of each of the protocols with the respective grouped protocols.

The medical imaging apparatus may include a magnetic resonance imaging apparatus. According to an aspect of another exemplary embodiment, there is provided a medical imaging apparatus including an interface configured to receive an input of a parameter for acquiring a medical image, and a controller configured to group protocols based on the input parameter, each of the protocols having one or more parameters for acquiring the medical image. The medical imaging apparatus further includes a display configured to display the grouped protocols.

The displayed protocols may have a table structure.

According to an aspect of another exemplary embodiment, there is provided a method of controlling a medical imaging apparatus, the method including receiving an input of a parameter for acquiring a medical image, and grouping protocols based on the input parameter, each of the protocols having one or more parameters for acquiring the medical image. The method further includes displaying the grouped protocols.

The displayed protocols have a tree structure.

A first protocol among the protocols may be grouped in a first column of the displayed protocols, and a second protocol among the protocols may be grouped in a second column of the displayed protocols, the second protocol referring to the first protocol.

The method may further include receiving an instruction to change a value of a parameter of a protocol among the protocols, and changing the value of the parameter of the protocol based on the instruction.

The method may further include displaying values of the input parameter in a first column of the displayed protocols, and each of the protocols may be grouped to correspond to a respective one among the values, in a second column of the displayed protocols.

According to an aspect of another exemplary embodiment, there is provided a medical imaging apparatus including a display configured to display parameters for acquiring a medical image, an interface configured to receive a selection of a parameter from the displayed parameters, and a controller configured to group protocols in respective values of the selected parameter, and control the display to display the selected parameter with the values and the grouped protocols.

BRIEF DESCRIPTION OF THE DRAWINGS

The above and/or other aspects will be more apparent by describing exemplary embodiments with reference to the accompanying drawings, in which:

FIG. 1 is a block diagram illustrating a magnetic resonance imaging apparatus according to an exemplary embodiment;

FIG. 2 is a view schematically illustrating an external appearance of the magnetic resonance imaging device of FIG. 1;

FIG. 3 is a view illustrating a division of a space, in which a target object is placed, along the X, Y and Z axes, according to an exemplary embodiment;

FIG. 4 is a view illustrating a structure of a magnet assembly and a structure of a gradient magnetic field coil of FIG. 1;

FIG. 5 is a diagram illustrating a pulse sequence regarding operations of respective gradient coils of the gradient magnetic field coil of FIG. 4;

FIG. 6 is a view illustrating a screen of a display on which a protocol list is displayed, according to an exemplary embodiment;

FIG. 7 is a view schematically illustrating information about image parameters included in a plurality of protocols, according to an exemplary embodiment;

FIGS. 8, 9, 10, and 11 are views illustrating protocols classified according to image parameters in a tress structure, according to exemplary embodiments;

FIGS. 12 and 13 are views illustrating a method of changing parameter values of one or more protocols that are classified based on an image parameter C1 in a tree structure, according to exemplary embodiments;

FIG. 14 is a view illustrating a method of changing parameter values of one or more protocols that are classified based on the image parameter C1 in a tree structure, according to another exemplary embodiment;

FIG. 15 is a view illustrating a method of changing parameter values of one or more protocols that are classified based on an image parameter C3 in a tree structure, according to an exemplary embodiment;

FIGS 16, 17, and 18 are views illustrating a screen on which a protocol list is displayed, according to exemplary embodiments;

FIGS. 19 and 20 are views illustrating a screen of a display showing a protocol list in which grouped protocols are arranged, according to exemplary embodiments; and

FIG. 21 is a flowchart illustrating a method of controlling a magnetic resonance imaging apparatus, according to an exemplary embodiment.

DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions may not be described in detail because they would obscure the description with unnecessary detail.

It will be understood that the terms "comprises" and/or "comprising" used herein specify the presence of stated features or components, but do not preclude the presence or addition of one or more other features or components. In addition, the terms such as "unit," "-er (-or)," and "module" described in the specification refer to an element for performing at least one function or operation, and may be implemented in hardware, software, or the combination of hardware and software.

A diagnosis apparatus to which the technology of a medical imaging apparatus and a control method thereof according to exemplary embodiments may represent one among a X-ray imaging apparatus, an X-ray fluoroscopic apparatus, a computed tomography (CT) scanner, a magnetic resonance imaging (MRI) apparatus, a positron emission tomography (PET) apparatus, and a ultrasound imaging apparatus. Although the following description is made in relation to a magnetic resonance imaging apparatus, the exemplary embodiments are not limited thereto.

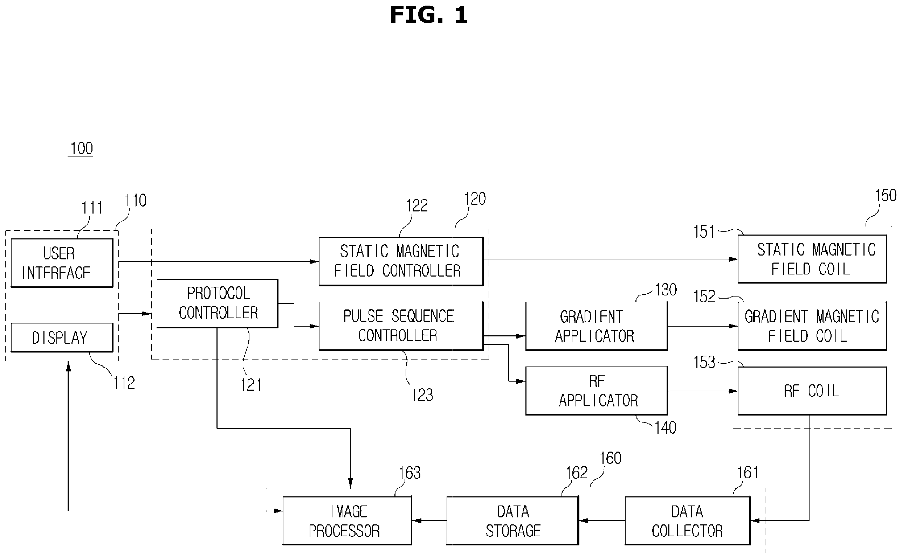

FIG. 1 is a block diagram of a magnetic resonance imaging device according to an exemplary embodiment. Hereinafter, the operation of a magnetic resonance imaging device 100 will be schematically described with reference to FIG. 1.

With reference to FIG. 1, the magnetic resonance imaging device 100 in accordance with an exemplary embodiment includes a magnet assembly 150 forming a magnetic field and generating resonance of atomic nuclei, a controller 120 controlling the operation of the magnet assembly 150, and an image processor 160 receiving an echo signal, i.e., a magnetic resonance signal, generated from the atomic nuclei and generating a magnetic resonance image.

The magnet assembly 150 includes a static magnetic field coil 151 forming a static magnetic field in the inner space thereof, a gradient magnetic field coil 152 generating gradients in the static magnetic field and thus forming gradient magnetic fields, and a radio frequency (RF) coil 153 exciting the atomic nuclei by applying an RF pulse and receiving an echo signal from these atomic nuclei. That is, when a target object is located in the inner space of the magnet assembly 150, the static magnetic field, the gradient magnetic fields and the RF pulse are applied to the target object, and thus, atomic nuclei of the target object are excited and an echo signal is generated from the atomic nuclei.

The controller 120 includes a protocol controller 121 determining an image parameter based on a protocol selected by a user, a static magnetic field controller 122 controlling the intensity and direction of the static magnetic field formed by the static magnetic field coil 151 according to the determined image parameter, and a pulse sequence controller 123 controlling the gradient magnetic field coil 152 and the RF coil 153 based on the pulse sequence by designing a pulse sequence.

The protocol controller 121, the static magnetic field controller 122, and the pulse sequence controller 123 each may include a memory storing a program and data for performing a respective function and a processor performing the respective function.

According to exemplary embodiments, the protocol controller 121, the static magnetic field controller 122 and the pulse sequence controller 123 may be implemented by respective memories and processors, or may be implemented by a single memory and a single processor.

The magnetic resonance imaging apparatus 100 includes a gradient applicator 130 applying a gradient signal to the gradient magnetic field coil 152 and an RF applicator 140 applying an RF signal to the RF coil 153, and the pulse sequence controller 123 may control the gradient applicator 130 and the RF applicator 140 to adjust the gradient magnetic fields formed in the inner space of the magnet assembly 150 and the RF applied to the atomic nuclei.

The RF coil 153 is connected to the image processor 160, and the image processor 160 includes a data collector 161 collecting data regarding a spin echo signal, i.e., the magnetic resonance signal, generated from the atomic nuclei, a data storage 162 storing data received by the data collector 161, and a data processor 163 generating a magnetic resonance image by processing the data stored in the data storage 162.

The data collector 161 may include a pre-amplifier amplifying the magnetic resonance signal received by the RF coil 153, a phase detector receiving the magnetic resonance signal transmitted from the pre-amplifier and then detecting a phase, and an analog-to-digital (A/D) converter converting an analog signal acquired through phase detection into a digital signal. Further, the data collector 161 transmits the magnetic resonance signal converted into the digital signal to the data storage 162.

A data space constructing a two-dimensional (2D) Fourier space is formed in the data storage 162, and when storage of overall data, scanning of which has been completed, is completed, the data processor 163 reconfigures the image of the target object by performing 2D inverse Fourier transform upon data in the 2D Fourier space. The reconfigured image is displayed on a display 112.

The data storage 162 may be implemented by a memory that stores programs and data for the data processor 163 to reconfigure an image, and the data processor 163 may include a processor that generates a control according to the programs and data stored in the memory.

In addition, according to an exemplary embodiment, the image processor 160 may be omitted. For example, the image processor 160 may be integrated into the controller 120 described above. Further, the magnetic resonance imaging apparatus 100 in accordance with an exemplary embodiment includes a user interface 110, and may thus receive control instructions regarding the overall operation of the magnetic resonance imaging apparatus 100 from a user and receive instructions regarding a scan sequence from the user and generate a pulse sequence thereby.

The user interface 110 includes a user interface 111 allowing a user to operate a system, and the display 112 displaying a control state and the image generated by the image processor 160 to allow the user to diagnose the health state of the target object.

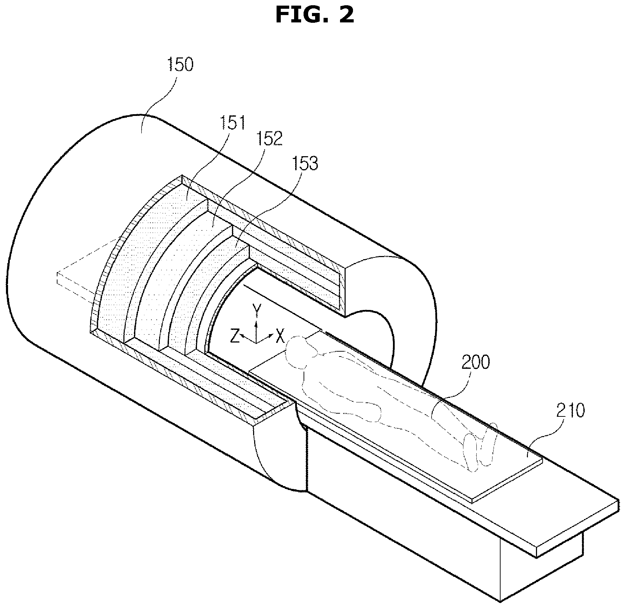

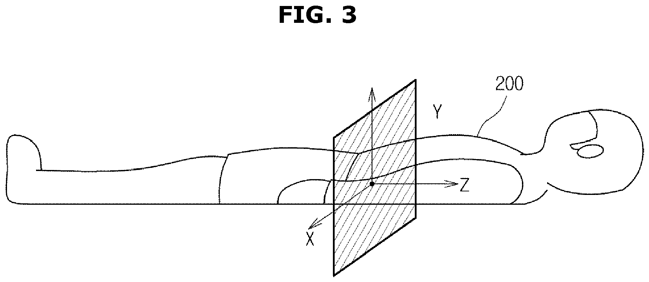

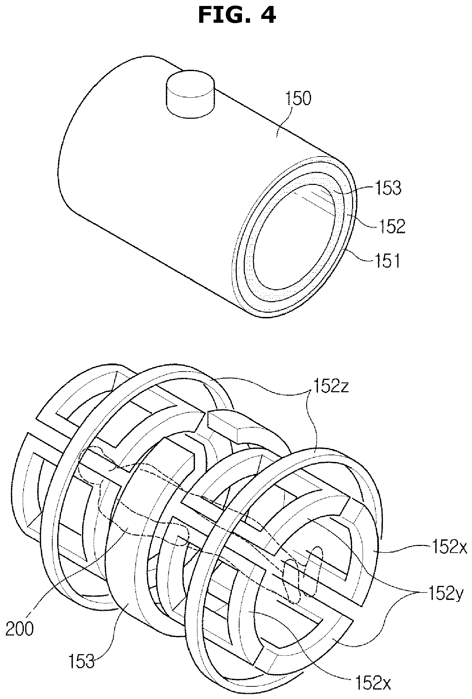

FIG. 2 is a view schematically illustrating an external appearance of the magnetic resonance imaging device of FIG. 1, FIG. 3 is a view illustrating a division of a space, in which a target object is placed, along the X, Y and Z axes, according to an exemplary embodiment, and FIG. 4 is a view illustrating a structure of the magnet assembly and a structure of the gradient magnetic field coil of FIG. 1.

Hereinafter, the detailed operation of the magnetic resonance imaging device 100 in accordance with an exemplary embodiment will be described with reference to FIG. 1 that has been described above.

With reference to FIG. 2, the magnet assembly 150 has a cylindrical shape, the inner space of which is vacant, and may be referred to as a gantry or a bore. The inner space of the magnet assembly 100 is referred to as a cavity, and a transfer portion 210 transfers a target object 200 laid thereon to the cavity to acquire a magnetic resonance signal.

The magnet assembly 150 includes the static magnetic field coil 151, the gradient magnetic field coil 152, and the RF coil 153.

The static magnetic field coil 151 may be formed in a shape in which a coil is wound around the cavity, and when current is applied to the static magnetic field coil 151, a static magnetic field is formed at the inside of the magnet assembly 150, i.e., in the cavity.

The direction of the static magnetic field is parallel with the driving axis of the magnet assembly 150.

When the static magnetic field is formed in the cavity, atomic nuclei of atoms constituting the target object 20, i.e., hydrogen atoms, are arranged in the direction of the static magnetic field and execute precession in the direction of the static magnetic field. The precession speed of atomic nuclei may be represented as a precession frequency, and such a precession frequency may be referred to as a Larmor frequency and expressed by Equation 1 below.

[Equation 1]

$$\omega = \gamma B0$$

Here, $\omega$ is a Larmor frequency, $\gamma$ is a proportional constant, and B0 is the intensity of an external magnetic field. The proportional constant varies according to kinds of atomic nuclei, the unit of the intensity of the external magnetic field is tesla (T) or gauss (G), and the unit of the precession frequency is Hz.

For example, hydrogen protons have a precession frequency of 42.58 Mhz in the external magnetic field of 1 T and, among elements constituting a human body, hydrogen occupies the largest percentage. Thus, a magnetic resonance signal is acquired predominantly using precession of hydrogen protons during MRI.

The gradient magnetic field coil 152 generates gradients in the static magnetic field formed in the cavity, thus forming gradient magnetic fields.

As shown in FIG. 3, an axis running parallel with the lengthwise direction from the head to the feet of the target object 200, i.e., an axis running parallel with the direction of the static magnetic field, may be defined as the Z-axis, an axis running parallel with the lateral direction of the target object 200 may be defined as the X-axis, and an axis running parallel with the vertical direction in the space may be defined as the Y-axis.

To acquire 3D spatial information of the magnetic resonance signal, gradient magnetic fields in all directions of the X-axis, Y-axis and Z-axis are used. Therefore, the gradient magnetic field coil 152 includes 3 pairs of gradient coils.

As shown in FIG. 4, Z-axis gradient coils 152z include a pair of ring type coils, and Y-axis gradient coils 152y are located above and below the target object 200. Further, X-axis gradient coils 152x are located at the left and right sides of the target object 200.

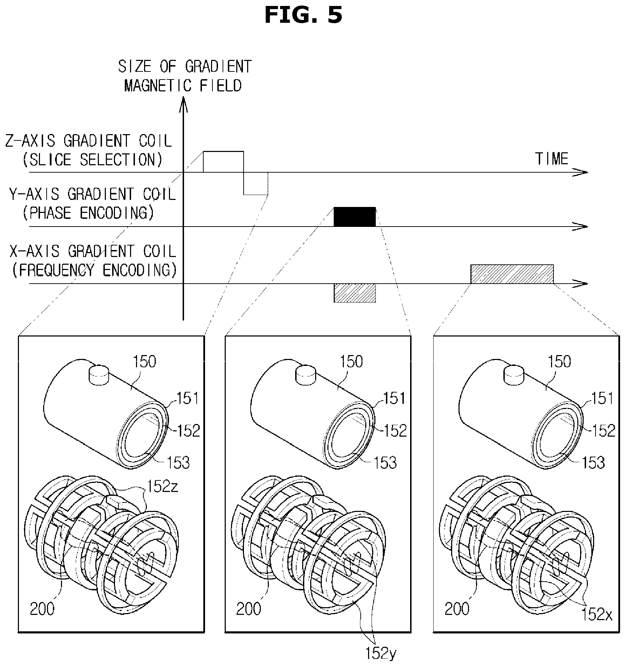

FIG. 5 is a diagram illustrating a pulse sequence regarding operations of the respective gradient coils of the gradient magnetic field coil of FIG. 4.

When direct currents having opposite polarities flow in the two Z-axis gradient coils 152z in opposite directions, the magnetic field is changed in the Z-axis direction and thus a gradient magnetic field is formed.

When the gradient magnetic field is formed by flow of current along the Z-axis gradient coils 152z for a designated time, the resonance frequency is changed to a higher frequency or a lower frequency according to the size of the gradient magnetic field. Then, when an RF signal corresponding to a position is applied through the RF coil 153, only protons of a slice corresponding to the position resonate. Therefore, the Z-axis gradient coils 152z are used in slice selection. As the gradient of the gradient magnetic field formed in the Z-axis direction is increased, a slice having a smaller thickness may be selected.

When the slice is selected through the gradient magnetic field formed by the Z-axis gradient coils 152z, all spins constituting the slice have the same frequency and the same phase and thus the respective spins are indistinguishable from one another.

At this time, when a gradient magnetic field in the Y-axis direction is formed by the Y-axis gradient coils 152y, the gradient magnetic field causes phase shift so that rows of the slice have different phases.

That is, when the Y-axis gradient magnetic field is formed, the phase of the spins of the row to which a large gradient magnetic field is applied is changed to a higher frequency, and the phase of the spins of the row to which a small gradient magnetic field is applied is changed to a lower frequency. When the Y-axis gradient magnetic field is removed, phase shift of the respective rows of the selected slice occurs, and thus the rows have different phases. Accordingly, the rows may be distinguished from one another. As described above, the gradient magnetic field formed by the Y-axis gradient coils 152y is used in phase encoding.

The slice is selected through the gradient magnetic field formed by the Z-axis gradient coils 152z, and the rows constituting the selected slice are distinguished from one another by different phases thereof through the gradient magnetic field formed by the Y-axis gradient coils 152y. However, the respective spins constituting each row have the same frequency and the same phase, and are thus indistinguishable from one another.

At this time, when a gradient magnetic field in the X-axis direction is formed by the X-axis gradient coils 152x, the X-axis gradient magnetic field causes the spins constituting each row to have different frequencies so that the respective spins are distinguishable from one another. As described above, the gradient magnetic field formed by the X-axis gradient coils 152x is used in frequency encoding.

As described above, the gradient magnetic fields formed by the Z-axis, Y-axis and X-axis gradient coils execute encoding of spatial positions of the respective spins, i.e., spatial encoding, through slice selection, phase encoding and frequency encoding.

Referring to FIGS. 1, 4, and 5, the gradient magnetic field coil 152 is connected to the gradient applicator 130, and the gradient applicator 130 applies a gradient waveform, i.e., a current pulse, to the gradient magnetic field coil 152 according to a control signal transmitted from the pulse sequence controller 122 and then generates gradient magnetic fields. Therefore, the gradient applicator 130 may be referred to as a gradient power supply, and include three drive circuits corresponding to the three pairs of gradient coils 152x, 152y and 152z of the gradient magnetic field coil 152. The detailed configuration of the operation of the gradient applicator 130 will be described later, and hereinafter, an electrical signal, such as a current or voltage pulse applied to the gradient applicator 130 to form gradient magnetic fields, may be referred to as a gradient waveform.

As described above, atomic nuclei arranged by the external magnetic field execute precession at the Larmor frequency, and the magnetization vector sum of several atomic nuclei may be represented as net magnetization M.

Measurement of a Z-axis component of the net magnetization M is impossible, and thus only Mxy may be detected. Therefore, to acquire a magnetic resonance signal, the net magnetization may be present on the X-Y plane through excitation of the atomic nuclei. To excite the atomic nuclei, an RF pulse tuned to the Larmor frequency of the atomic nuclei may be applied to the static magnetic field.

The RF coil 153 includes a transmission coil transmitting the RF pulse, and a reception coil receiving electromagnetic waves emitted from the excited atomic nuclei, i.e., a magnetic resonance signal. In addition, the reception coil is divided into a whole-volume coil that receives a magnetic resonance signal excited from the entire area of the target object and a local coil or a surface coil that receives a magnetic resonance signal excited from a part of the target object.

The following description will be made on the assumption the reception coil is a local coil that receives a magnetic resonance signal excited from a part of a target object.

The RF coil 153 is connected to the RF applicator 140, and the RF applicator 140 applies an RF signal to the RF coil 153 according to a control signal transmitted from the pulse sequence controller 122 so that the RF coil 153 may transmit the RF pulse to the inside of the magnet assembly 150.

The RF applicator 140 may include a modulation circuit modulating an RF signal into a pulse type signal, and an RF power amplifier amplifying the pulse type signal.

As a method to acquire a magnetic resonance signal from atomic nuclei, a spin echo pulse sequence is used. If the RF coil 153 applies RF pulses, when an RF pulse is transmitted one more time at a proper time interval $\Delta t$ after application of the first RF pulse, strong transverse magnetization of the atomic nuclei occurs after a time $\Delta t$ therefrom has elapsed, and a magnetic resonance signal may be acquired therefrom. This is referred to as a spin echo pulse sequence, and time taken to generate the magnetic resonance signal after application of the first RF pulse is referred to as echo time.

A flip degree of protons may be represented as an angle to which the protons move from the axis where the protons are located before flip, and be represented as a 90 degree RF pulse, a 180 degree RF pulse, etc. according to the flip degree of the protons.

A user may obtain a magnetic resonance image by using various image parameters. Depending on an image parameter selected by a user, a method of designing a pulse sequence in the pulse sequence controller 123 or a method of reconstructing an image in the image processor may be changed, which causes a magnetic resonance image to be differently generated. The image parameter includes an image photography technique and an image processing technique.

A set of one or more image parameters is referred to as a protocol, and one or more protocols may be displayed in the form of a protocol list on the display 112.

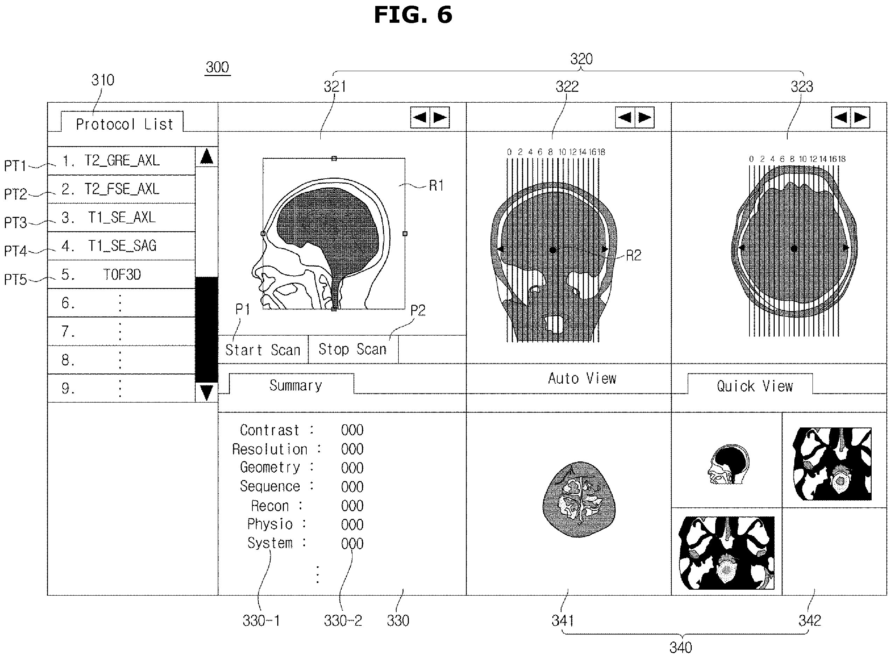

FIG. 6 is a view illustrating a screen of a display on which a protocol list is displayed, according to an exemplary embodiment.

With reference to FIG. 6, a protocol list 310 is displayed on a screen 300 of the display 112, and the protocol list 310 may display names of one or more protocols including protocols PT1 to PT5. The names of the protocols PT1 to PT5 may represent parameter values of image parameters included in the protocols PT1 to PT5, respectively, and may be randomly changed by a user through the user interface 111.

For example, the name of a first protocol PT1 may indicate a T2 weighted image using a gradient recalled echo (GRE) parameter in an axial direction, and the name of a second protocol PT2 may indicate a T2 weighted image using a fast spin echo (FSE) parameter in an axial direction.

A user may select a protocol (e.g., the first protocol PT1) displayed on the protocol list 310 by using the user interface 111. If the user selects the first protocol PT1, in a section 330 displaying parameters 330-1 and parameter values 330-2, the parameter values 330-2 may be changed to parameter values included in the first protocol PT1, or in at least one of sections 321, 322 and 323 displaying views of a reference image of a section 320, the size or position of a localizing region R1 or the position of a localizing point R2 may be changed.

For example, each image parameter may represent a contrast, a resolution, a geometry, a sequence type, slice information, a photography direction, a default resolution, or an object referred to.

For example, the first protocol PT1 may include information indicating a Time Repetition (TR) of 600ms and a Time Echo (TE) of 24ms as parameter values for a contrast, information indicating "Axial direction" as a parameter value for a photography direction, and information indicating 448 as a parameter value for a default resolution, and when the first protocol PT1 is selected, the parameter section 330 may display information indicating a TR of 20ms, information indicating a TE of 6ms, information indicating a photography direction of "Axial direction", and information indicating a default resolution of 448.

In addition, when a user selects an image generation instruction icon PI by using the user interface 111, the protocol controller 121 may generate a control signal based on the image parameter included in the selected first protocol PT1 for at least one among the pulse sequence controller 123 and the image processor 160.

In this case, a magnetic resonance image automatically generated according to the control signal of the image processor 160 may be displayed on a section 340 (including sections 341 and 342 showing an auto view and a quick view, respectively, of the image) of the screen 300 of the display 112.

That is, as the control signal of the protocol controller 121 is generated, a magnetic resonance image corresponding to the first protocol PT1 is generated until a "Stop Scan" icon P2 is selected by the user, and the generated magnetic resonance image may be displayed on one or more of the sections 321, 322, and 323 of the screen 300 of the display 112.

For example, when the image generation instruction icon PI is selected, a magnetic resonance image is acquired in an axial direction according to a control signal of the protocol controller 121, the magnetic resonance image is subject to an image processing with a contrast of +25, and the image processed magnetic resonance image is displayed on one or more of the sections 321, 322, and 323.

The localizing region R1 or the localizing point R2 displayed on the one or more of the sections 321, 322, 323 may be an object referred to by another protocol PT2 to PT5.

For example, when a localizing region R1 corresponding to the first protocol PT1 is set in a first image section 321 and a localizing point R2 is set in a second image section 322, the localizing region R1 may be an object to be referred to by a second protocol PT2, and the localizing point R2 may be an object to be referred to by a third protocol PT3. That is, the second protocol PT2 may include an image parameter based on information about a region R1 within the first image section 321, and the third protocol PT3 may include an image parameter based on information about a point R2 within the second image section 322.

In this case, the first protocol PT1 may be a reference protocol of the second protocol PT2, and the second protocol PT2 may be a reference protocol of the third protocol PT3.

The display 112 may display the protocols PT1 to PT5 on the protocol list 310 not only in an arrangement but also in a classified form according to image parameters.

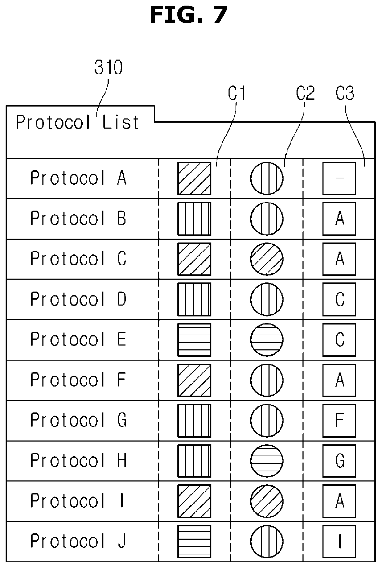

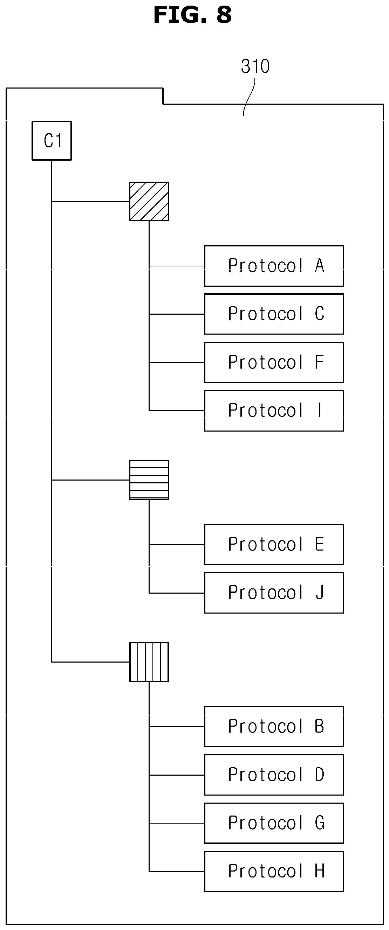

FIG. 7 is a view schematically illustrating information about image parameters included in a plurality of protocols, according to an exemplary embodiment, and FIGS. 8, 9, 10, and 11 are views illustrating protocols classified according to image parameters in a tree structure, according to exemplary embodiments.

With reference to FIG. 7, each protocol of the protocol list 310 includes parameter values corresponding to one or more image parameters, e.g., image parameters C1, C2 and C3. In this case, each protocol (for example, protocol A) may not include a parameter value corresponding to a parameter (for example, C3).

For convenience of description, the following description will be made in relation that protocol A has an oblique stripe square as a parameter value of an image parameter C1, a vertical stripe circle as a parameter value of an image parameter C2, and Null as a parameter value of an image parameter C3 (that is, having no parameter value for C3); protocol B has a vertical stripe square as a parameter value of the image parameter C1, a vertical stripe circle as a parameter value of the image parameter C2, and reference protocol-A as a parameter value of the image parameter C3; protocol C has an oblique stripe square as a parameter value of the image parameter C1, an oblique stripe circle as a parameter value of the image parameter C2, and reference protocol-A as a parameter value of the image parameter C3; protocol D has a vertical stripe square as a parameter value of the image parameter C1, a vertical stripe circle as a parameter value of the image parameter C2, and reference protocol-C as a parameter value of the image parameter C3; protocol E has a horizontal stripe square as a parameter value of the image parameter C1, a horizontal stripe circle as a parameter value of the image parameter C2, and reference protocol-C as a parameter value of the image parameter C3; protocol F has an oblique stripe square as a parameter value of the image parameter C1, a vertical stripe circle as a parameter value of the image parameter C2, and reference protocol-A as a parameter value of the image parameter C3; protocol G has a vertical stripe square as a parameter value of the image parameter C1, a vertical stripe circle as a parameter value of the image parameter C2, and reference protocol-F as a parameter value of the image parameter C3; protocol H has a vertical stripe square as a parameter value of the image parameter C1, a horizontal stripe circle as a parameter value of the image parameter C2, and reference protocol-G as a parameter value of the image parameter C3; protocol I has an oblique stripe square as a parameter value of the image parameter C1, an oblique stripe circle as a parameter value of the image parameter C2, and reference protocol-A as a parameter value of the image parameter C3; and protocol J has a horizontal stripe square as a parameter value of the image parameter C1, a vertical stripe circle as a parameter value of the image parameter C2, and reference protocol-I as a parameter value of the image parameter C3.

In addition, the following description will be made in relation that the image parameter C1 represents a photography direction, the image parameter C2 represents a resolution, and the image parameter C3 represents a reference protocol.

For example, an oblique stripe square as a parameter value of the image parameter C1 may represent an Axial direction, a vertical stripe square as a parameter value of the image parameter C1 may represent a Sagittal direction, and a horizontal stripe square as a parameter value of the image parameter C1 may represent an Transverse direction; an oblique stripe circle as a parameter value of the image parameter C2 may represent a resolution of 300, a vertical stripe circle as a parameter value of the image parameter C2 may represent a resolution of 200, and a horizontal stripe circle as a parameter value of the image parameter C2 may represent a resolution of 100. However, the exemplary embodiments are not limited to the image parameters and parameter values described above.

The magnetic resonance imaging apparatus 100 in accordance with an exemplary embodiment may group one or more of the protocols A to J displayed on the protocol list 310 based on an image parameter, and display the grouped one or more protocols A to J on the display 112.

With reference to FIG. 8, the protocol controller 121 of the magnetic resonance imaging apparatus 100 may group the protocols A to J based on the image parameter C1.

In this case, the protocol controller 121 may define protocols A, C, F, and I having a parameter value of an oblique stripe square as one parameter group, define protocols E and J having a parameter value of a horizontal stripe square as another parameter group, and define protocols B, D, G and H having a parameter value of a vertical stripe square as another parameter group.

A parameter group represents a set of protocols that have the same parameter value.

The display 112 may display one or more protocols (A to J) according to parameter groups defined by the protocol controller 121 on the protocol list 310.

A user may intuitively recognize the one or more protocols classified based on the image parameter C1, and select one among the protocols (A to J).

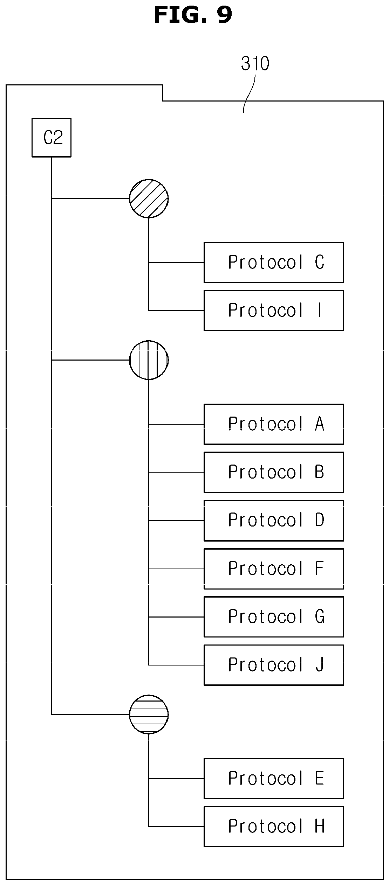

With reference to FIG. 9, the protocol controller 121 of the magnetic resonance imaging apparatus 100 may group the protocols A to J based on the image parameter C2.

In this case, the protocol controller 121 may define protocols C and I having a parameter value of an oblique stripe circle as one parameter group, define protocols A, B, D, F, G and J having a parameter value of a vertical stripe circle as another parameter group, and define protocols E and H having a parameter value of a vertical stripe circle as another parameter group.

The display 112 may display one or more protocols (A to J) according to parameter groups defined by the protocol controller 121 on the protocol list 310.

A user may intuitively recognize the one or more protocols classified based on the image parameter C2, and select one among the protocols (A to J).

The image parameter serving as a criterion for classifying protocols may be directly selected by a user through the user interface 111. For example, when a user selects the image parameter C1, the protocol controller 121 may classify one or more protocols (A to J) based on the image parameter C1, and when a user selects the image parameter C2, the protocol controller 121 may classify one or more protocols (A to J) based on the image parameter C2.

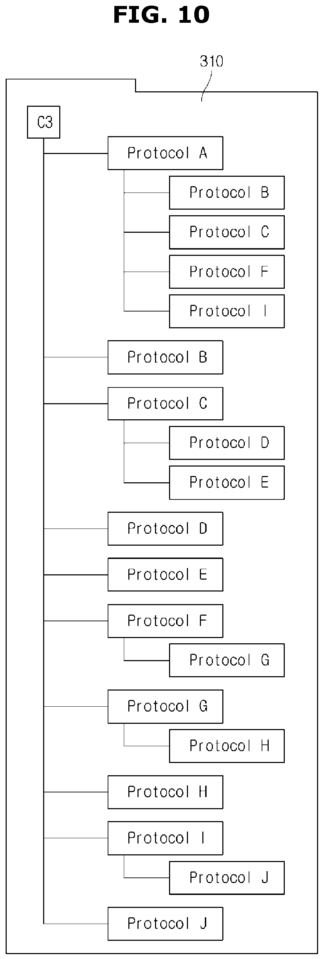

With reference to FIG. 10, the protocol controller 121 of the magnetic resonance imaging apparatus 100 in accordance with an exemplary embodiment may group the protocols A to J based on the image parameter C3. The following description will be made in relation that the image parameter C3 is a reference protocol.

The protocol controller 121 may define protocols B, C, F and I having a parameter value of a reference protocol 'protocol A' as one parameter group, define protocols D and E having a parameter value of a reference protocol 'protocol C' as another parameter group, define protocol G having a parameter value of a reference protocol 'protocol F 'as another parameter group, define protocol H having a parameter value of a reference protocol 'protocol G' as another parameter group, and define protocol J having a parameter value of a reference protocol 'protocol I' as another parameter group.

The display 112 lists protocols A to J in a first column on the protocol list 310 according to a control signal of the protocol controller 121, and displays protocols that refer to the protocols A to J to correspond to the parameter groups defined by the protocol controller 121.

A user may intuitively recognize the one or more protocols A to J classified based on the reference protocol, and select one among the protocols A to J.

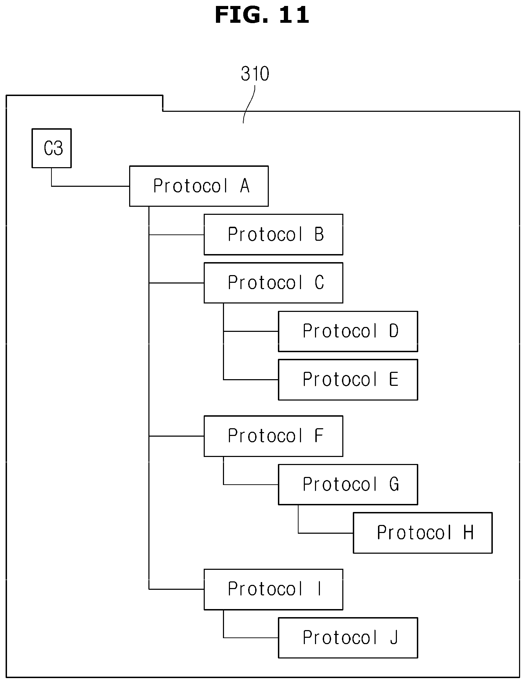

When the image parameter C3 is a reference protocol, the protocol controller 121 of the magnetic resonance imaging device 100 in accordance with an exemplary embodiment may arrange one or more protocols A to J in a tree structure. FIG. 11 is a view illustrating a protocol list including one or more protocols arranged in a tree structure.

Referring to FIG. 11, when protocol A is defined as the highest level protocol (the first column), the protocol controller 121 defines protocols B, C, F and I referring to protocol A as lower level protocols of protocol A (the second column), defines protocols D and E referring to protocol C as lower level protocols of protocol C (the third column), defines protocol G referring to protocol F as a lower level protocol of protocol F (the third column), and defines protocol J referring to protocol I as a lower level protocol of protocol I (the third column). Further, the protocol controller 121 defines protocol H referring to protocol G existing in the third column as a lower level protocol of protocol G (the fourth column).

The display 112 lists protocols defined as the first to fourth columns to be distinguished by the respective columns according to a control signal of the protocol controller 121. In this case, the protocols referred to may be indicted as shown in FIG. 11.

With reference to FIG. 11, when protocols A to J are grouped based on the image parameter C3 and an image generation instruction according to protocol A is input by a user (for example, when a scan initiation icon PI is selected after icon PT1 shown in FIG. 6 is selected), a magnetic resonance image generated according to protocol A is displayed on the section 320 on the screen 300 of the display 112 that are configured to display a magnetic resonance image.

In this case, lower protocols of protocol A (that is, protocols B, C, F and I) generate different magnetic resonance images based on the generated magnetic resonance image of protocol A. When protocol B and the first image section 321 are selected according to a user's manipulation (for example, upon a drag and drop of an icon corresponding to protocol B on the first image section 321), information about a region or point that is referred to by protocol B in the magnetic resonance image of protocol A being displayed in the first image section 321 may be displayed on the first image section 321.

In addition, when protocol B and the second image section 322 are selected according to a user's manipulation (for example, upon a drag and drop of a PT2 icon on the second image section 322), information about a region or point that is referred to by protocol B in the magnetic resonance image of protocol A being displayed in the second image section322 may be displayed on the second image section 322.

Generations of the remaining lower protocols of protocol A (that is, protocols C, F and I) may be achieved in the same manner as the above.

In addition, when an image generation instruction according to protocol C is input by a user, a magnetic resonance image generated according to protocol C is displayed on the section 320 on the screen 30 of the display 112 that are configured to display a magnetic resonance image.

In this case, lower protocols of protocol C (that is, protocols D, E) generates different magnetic resonance images based on the generated magnetic resonance image of protocol C. When protocol D is selected according to a user's manipulation (for example, upon a drag and drop of an icon corresponding to protocol D on the first image section 321), information about a region or point that is referred to by protocol C in the magnetic resonance image of protocol C being displayed in the first image section 321 may be displayed on the first image section 321.

Generation of the remaining lower protocol of protocol C (that is, protocol E) may be achieved in the same manner as the above.

The magnetic resonance imaging apparatus 100 in accordance with another exemplary embodiment may receive an instruction regarding changing a parameter value of an image parameter included in each protocol from a user, and may change a parameter value according to the received instructions.

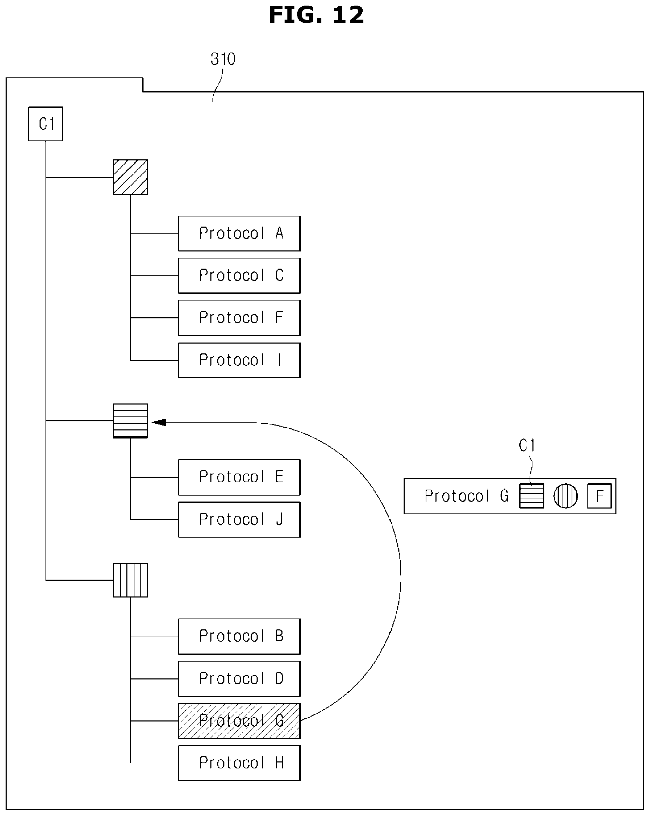

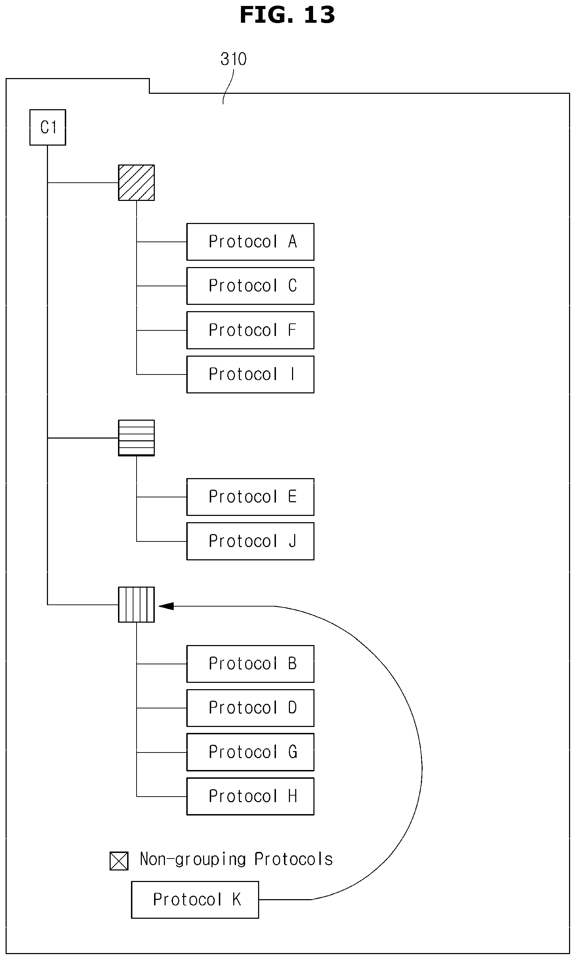

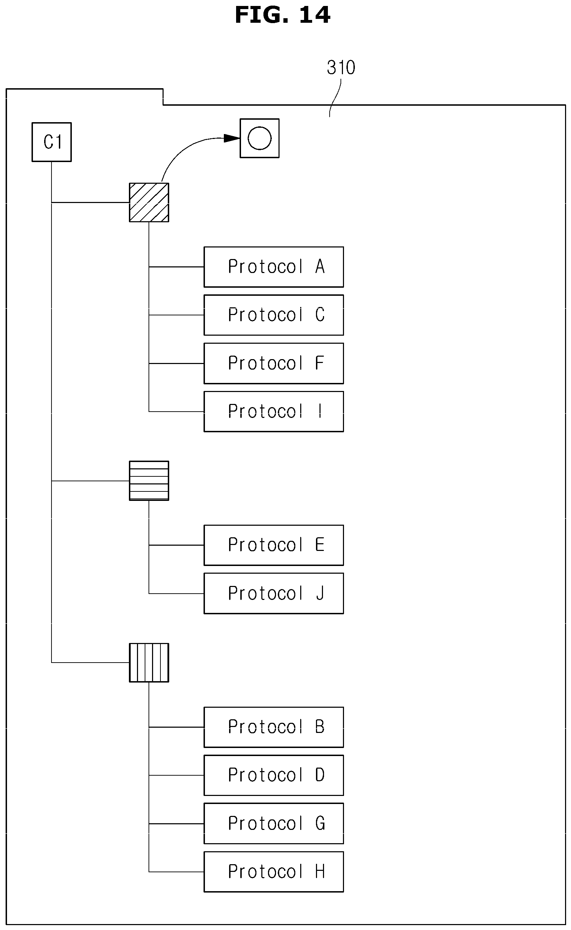

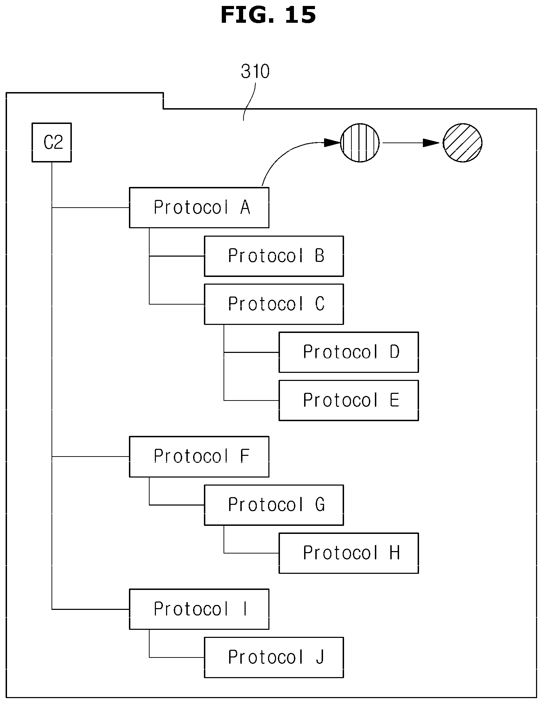

FIGS. 12 and 13 are views illustrating a method of changing parameter values of one or more protocols that are classified based on the image parameter C1 in the tree structure, according to exemplary embodiments, and FIG. 14 is a view illustrating a method of changing parameter values of one or more protocols that are classified based on the image parameter C1 in the tree structure, according to another exemplary embodiment. FIG. 15 is a view illustrating a method of changing parameter values of one or more protocols that are classified based on the image parameter C3 in the tree structure, according to an exemplary embodiment.

With reference to FIG. 12, a user looks up the protocol list 310 displayed on the display 112, and moves a parameter group to which at least one protocol belongs by manipulating the user interface 111, thereby changing a parameter value of an image parameter included in the protocol (for example, image parameter C1).

For example, when a parameter group to which protocol G belongs has a parameter value of a vertical stripe square, a user may drag and drop protocol G onto a horizontal stripe square by manipulating the user interface 111, so that the protocol controller 121 may change a parameter value of image parameter C1 of protocol G from the vertical stripe square to the horizontal stripe square.

In addition, with reference to FIG. 13, with respect to protocol K having no parameter value for image parameter C1, the protocol controller 121 may store a parameter value for the image parameter C1 according to a user's manipulation.

For example, when there is no parameter group to which protocol K belongs, a user may drag and drop protocol K onto a vertical strip square by manipulating the user interface 111, so that the protocol controller 121 may designate a parameter value of protocol K for the image parameter C1 as the vertical strip square.

In addition, with reference to FIG. 14, a user looks at the protocol list 310 displayed on the display 112 and directly changes a property of a parameter group to which at least one protocol belongs by manipulating the user interface 111, thereby collectively changing parameter values of the at least one parameter belonging to the parameter group.

For example, when a user changes a property of an oblique stripe square group into a circle-containing square by manipulating the user interface 111, the protocol controller 121 may collectively change parameter values of protocols A, C, F and I, belonging to the oblique stripe square, with respect to image parameter C1, into the circle-containing square. That is, the changing of a parameter value of a parameter group is inherited to one or more protocols belonging to the parameter group.

Similarly, with reference to FIG. 15, when one or more protocols arranged based on image parameter C2 are displayed to a user and a parameter value of a protocol is changed, the change may lead to change in a parameter value of a lower protocol of the protocol whose parameter value is changed.

For example, when one or more protocols A to J are arranged in a tree structure based on protocol A on the protocol list 310, a user may change a parameter value of protocol A with respect to image parameter C2 from the vertical stripe circle to the oblique stripe circle by manipulating the user interface 111.

In addition, according to the user's manipulation, the protocol controller 121 may change parameter values of protocols B,C, F and I that are lower protocols of protocol A, parameter values of protocols D and E that are lower protocols of protocol C, a parameter value of protocol G that is a lower protocol of protocol F, a parameter value of protocol H that is a lower protocol of protocol G, and a parameter value of protocol J that is a lower protocol of protocol I, into oblique stripe circles.

When a user changes a parameter value of protocol F with respect to image parameter C2 into an oblique stripe circle by manipulating the user interface 111, the protocol controller 121 may change a parameter value of protocol G that is a lower protocol of protocol F and a parameter value of protocol H that is a lower protocol of protocol G into oblique stripe circles. However, a parameter value of protocol A that is a upper protocol of protocol F with respect to image parameter C2 is not changed.

That is, the changing of the parameter value has an influence only on the lower protocol. Although the above description has been made in relation that a protocol list is displayed in a tree structure, the display 112 may display the protocol list in various structures.

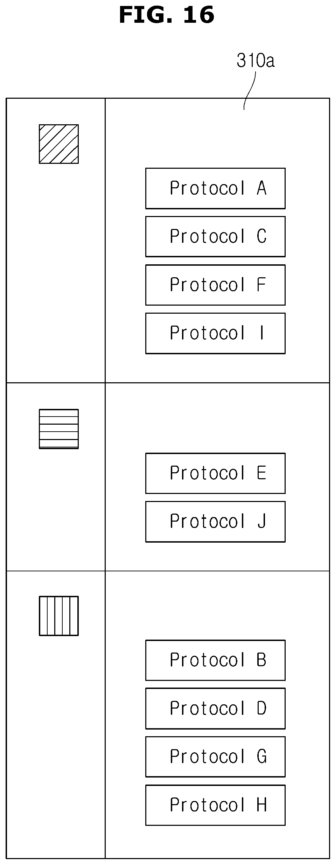

FIGS. 16, 17, and 18 are views illustrating a screen on which a protocol list is displayed, according to exemplary embodiments.

With reference to FIG. 16, the display 112 in accordance with an exemplary embodiment displays a protocol list 310a in a table structure. The following description will be made in relation that the magnetic resonance imaging apparatus 100 receives selection of an image parameter from a user, and classifies a plurality of protocols based on the received image parameter.

For example, the display 112 in accordance with an exemplary embodiment may arrange one or more parameter groups in a first column in the table, and arrange one or more protocols belonging to each of the parameter groups in a second column in the table.

In detail, when a user selects "square" as an image parameter, the display 112 may display a first group having a parameter value of an oblique stripe square, a second group having a parameter value of a horizontal stripe squire, and a third group having a parameter value of a vertical stripe squire in the first column in the form of a text or a diagram.

In addition, protocols A, C, F and I belonging to the first group are displayed to correspond to the first group in the second column, protocols E and J belonging to the second group are displayed to correspond to the second group in the second column, and protocols B, D, G and H belonging to the third group are displayed to correspond to the third group in the second column.

On the contrary, one or more parameter groups may be arranged in a first row of the table, and one or more protocols belonging to each of the parameter groups may be arranged in a second row of the table.

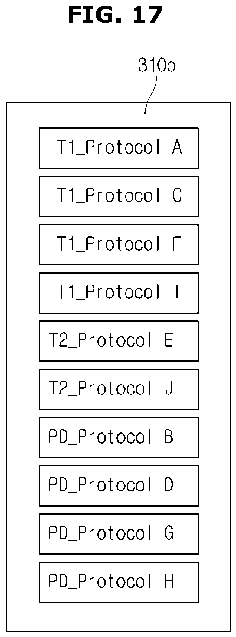

In addition, with reference to FIG. 17, the display 112 in accordance with another exemplary embodiment displays a protocol list 310b having protocols arranged in the order of parameter groups, in which the protocol list 310b displays a protocol name changed by the protocol controller to include information about a parameter value.

For example, when a user selects an image parameter "contrast" and protocols A and C include parameter values T1 (TR: 600ms and TE: 24ms), the protocol controller 121 may change a protocol name corresponding to protocol A from "Protocol A" into "T1_Protocol A".

In addition, the protocol controller 121 may change a protocol name corresponding to protocol C from "Protocol C" into "T1_Protocol C". As with other protocols, the protocol names may be changed in the same manner as the above to include information about parameter values.

In this case, the protocol list 310b sequentially arranges protocols A, C and F (the first group) that include T1 parameter values, sequentially arranges protocols E and J (the second group) that include another contrast parameter value T2 (TR: 4000ms and TE: 72ms), and then sequentially arranges protocols B, D, G and H (the third group) that include another contrast parameter value PD (TR: 3000ms and TE: 34ms).

The order of parameter groups arranged may be previously stored, for example, in the order of alphabetical indexes.

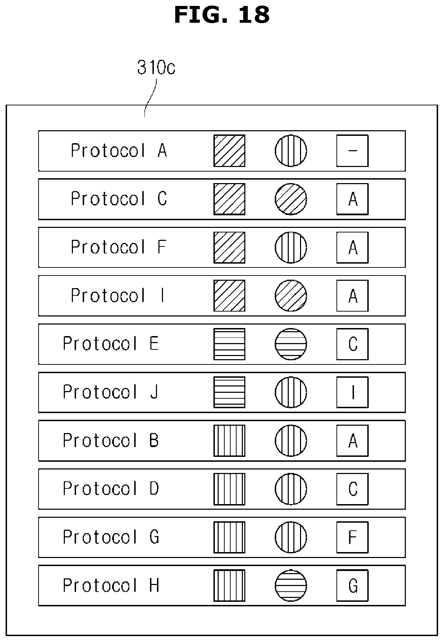

With reference to FIG. 18, the display 112 in accordance with another exemplary embodiment displays a protocol list 310c in which protocols are arranged in the order of parameter groups while displaying information about a parameter value included in each protocol together with a protocol name.

In this case, the display 112 may display a protocol value by using a color or diagram that represents each protocol.

For example, when a user selects "square" as an image parameter, and protocols A, C, F and I (the first group) include T1 as a parameter value for a contrast, the display 112 may display an oblique stripe square representing T1 together with protocol names (Protocol A, Protocol C, Protocol F, and Protocol I).

In addition, when protocols E and J (the second group) following the first group include T2 as a parameter value for a contrast, the display 112 may display a horizontal stripe square representing T2 together with protocol names (Protocol E and Protocol J).

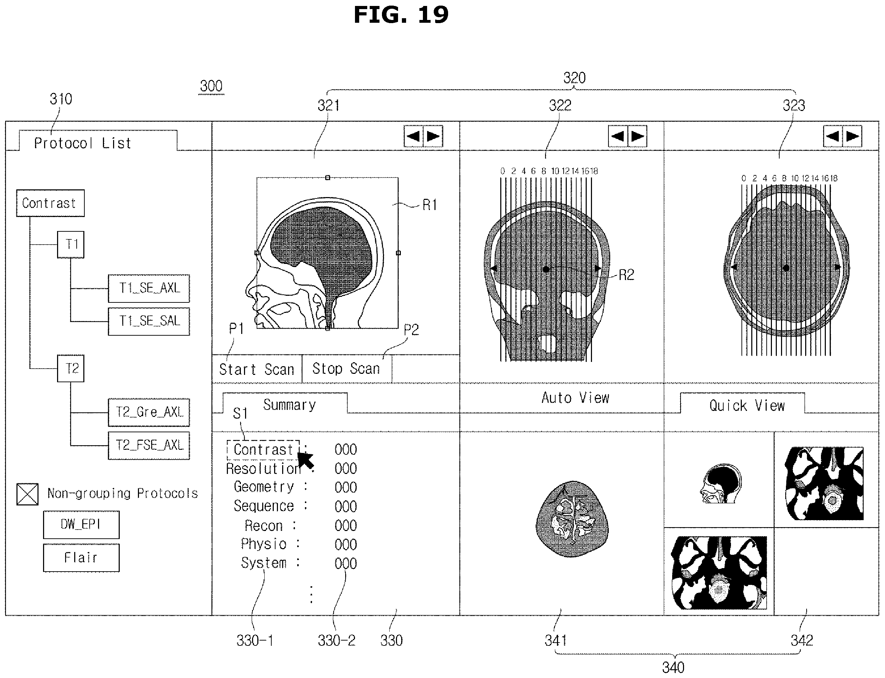

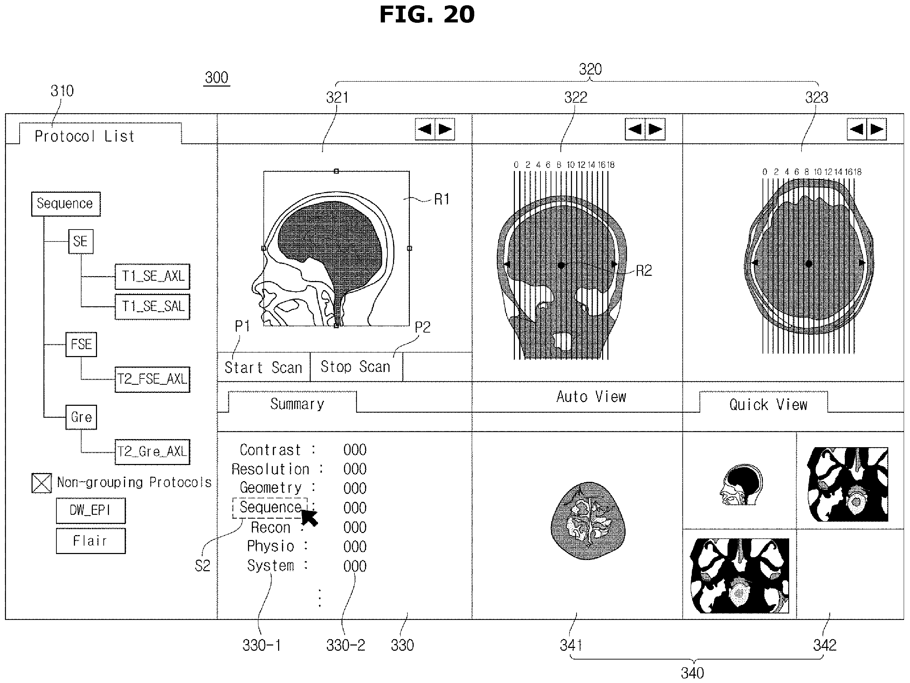

In addition, when protocols B, D, G and H (the third group) following the second group include T3 as a parameter value for a contrast, the display 112 may display a vertical stripe square representing T3 together with protocol names (Protocol B, Protocol D, Protocol G and Protocol H). A criterion for determining a parameter group (that is, a criterion for classifying protocols) may vary with a user's selection of parameter, and the selection of a parameter will be described with reference to FIGS. 19 and 20 in detail.

The color and diagram representing each protocol value are not limited to the above described example.

The protocol list 310 arranging the grouped protocols in accordance with exemplary embodiments may be displayed on a section on the screen of the display 112 of the magnetic resonance imaging apparatus.

FIGS. 19 and 20 are views illustrating a screen of a display showing a protocol list in which grouped protocols are arranged, according to exemplary embodiments. Although a protocol list of FIGS. 19 and 20 is illustrated as having protocols classified in a tree structure, the protocols may be classified in various structures.

With references to FIGS. 19 and 20, a criterion for classifying protocols may be selected (that is, a criterion for determining a parameter group) by a users' manipulation or a program previously stored in the protocol controller 121, and according to the selected criterion for classifying protocols, grouped protocols may be displayed on the protocol list 310.

For example, when a use selects a contrast S1 as a criterion for classifying protocols, the protocol list 310 may display protocols grouped based on the contrast.

With reference to FIG. 20, when a user selects sequence type S2 as a criterion for classifying protocols, the protocol list 310 may display protocols grouped based on the sequence type.

The criterion for classifying protocols is not limited to the contrast and the sequence type as described above, and may be provided as any one among various image parameters.

Hereinafter, a method of controlling the magnetic resonance imaging apparatus 100 in accordance with an exemplary embodiment will be described.

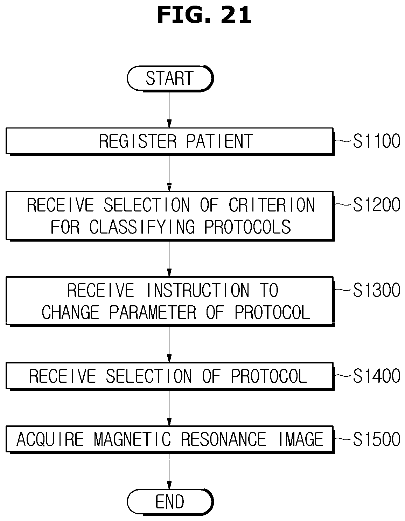

FIG. 21 is a flowchart illustrating a method of controlling a magnetic resonance imaging apparatus, according to an exemplary embodiment. In the description of FIG. 21, the same reference numerals are used to refer to the parts, which are the same as those described with reference to FIGS. 1 to 20.

In operation S1100, the magnetic resonance imaging apparatus 100 registers information about a patient according to a user's manipulation using the user interface 111. In operation S1200, the display 112 displays a screen that allows a user to select a criterion for classifying protocols by manipulating the user interface 111, and the user interface 111 receives a user selection of a criterion for classifying the protocols.

For example, when an image parameter is selected as a criterion for classifying protocols, protocols may be grouped according to parameter values for the selected image parameter.

In this case, the selecting of a criterion for classifying protocols may be not achieved only by a user's manipulation, but also achieved by a program previously stored in the protocol controller 121 in an automatic manner.

The criterion for classifying protocols may be one among various image parameters included in protocols.

In operation S1300, the protocol controller 121 receives a parameter value change instruction to change a parameter of a protocol from a user through the user interface 111.

The parameter value change instruction may be an instruction to move or designate a parameter group to which a protocol belongs as shown in FIGS. 12 and 13, or an instruction to directly change a parameter value as shown in FIGS. 14 and 15.

A process of receiving a parameter value change instruction for a protocol may be omitted.

In operation S1400, the protocol controller 121 receives a selection of a protocol from a user through the user interface 111, and according to the selected protocol, parameter values of the pulse sequence controller 123 and the image processor 163 may be changed. In this case, the changed parameter values may be displayed on the display 112.

In operation S1500, the magnetic resonance imaging apparatus 100 acquires a magnetic resonance image based on the selected protocol. For example, the protocol controller 121, upon receiving a magnetic resonance image generation instruction from the user interface 111, may control the pulse sequence controller 123 to generate a pulse sequence according to the changed parameter value, or control the data processor 163 to perform an image processing on the photographed magnetic resonance image according to the changed parameter value.

In addition, the exemplary embodiments may also be implemented through computer-readable code and/or instructions on a medium, e.g., a computer-readable medium, to control at least one processing element to implement any above-described exemplary embodiments. The medium may correspond to any medium or media that may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

The foregoing exemplary embodiments are examples and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

**Claims**

1. A medical imaging apparatus comprising:

   a display (112) configured to display a list of protocols (310), each of the protocols having one or more parameters for acquiring a medical image;
   an interface (111) configured to receive an input of a parameter for acquiring the medical image; and
   a controller (121) configured to group the protocols based on the input parameter, and control the display to display the list of the grouped protocols,
   the interface (111) is further configured to receive an instruction to change a value of a parameter of one or more protocols among the protocols, and the controller (121) is further configured to change the value of the parameter of the one or more protocols based on the instruction;
   **characterised in that**
   the instruction to change a value of a parameter of the one or more protocols comprises an instruction to move the one or more protocols from a parameter group to which the one or more protocols belong into another parameter group, and/or an instruction to change a property of a parameter group to which the one or more protocols belong.

2. The medical imaging apparatus of claim 1, wherein the displayed list of the grouped protocols has a tree structure.

3. The medical imaging apparatus of claim 1, wherein the display is further configured to display, in the displayed list of the protocols, the one or more parameters of each of the protocols with the respective protocols.

4. The medical imaging apparatus of claim 1, wherein the one or more parameters of each of the protocols comprises at least one among a contrast, a resolution, a geometry, a sequence type, slice information, a photography direction and an object referred to, and
   the input parameter comprises one among the contrast, the resolution, the geometry, the sequence type, the slice information, the photography direction and the object referred to.

5. The medical imaging apparatus of claim 1, wherein a first protocol among the protocols is grouped in a first column of the displayed list of the grouped protocols, and
   a second protocol among the protocols is grouped in a second column of the displayed list of the grouped protocols, the second protocol referring to the first protocol.

6. The medical imaging apparatus of claim 1, wherein the interface is further configured to receive a selection of a protocol from the protocols, and
the controller is further configured to generate a pulse sequence and perform an image processing, based on the selected protocol.

7. The medical imaging apparatus of claim 1, wherein the controller is further configured to control the display to display values of the input parameter in a first column of the displayed list of the grouped protocols, and
each of the protocols is grouped to correspond to a respective one among the values, in a second column of the displayed list of the grouped protocols.

8. The medical imaging apparatus of claim 1, wherein the interface is further configured to receive an instruction to change a value among the values, and
the controller is further configured to change the value for the grouped protocols to correspond to the value, based on the instruction.

9. The medical imaging apparatus of claim 1, wherein the display is further configured to display, in the displayed list of the grouped protocols, the one or more parameters of each of the protocols with the respective grouped protocols.

10. A method of controlling a medical imaging apparatus, the method comprising:

   receiving an input of a parameter for acquiring a medical image;
   grouping protocols based on the input parameter, each of the protocols having one or more parameters for acquiring the medical image; and
   displaying the grouped protocols,
   the method further comprising receiving an instruction to change a value of a parameter of one or more protocols among the protocols, and
   changing the value of the parameter of the one or more protocols based on the instruction;
   **characterised in that**
   the instruction to change a value of a parameter of the one or more protocols comprises an instruction to move the one or more protocols from a parameter group to which the one or more protocols belong into another parameter group, and/or an instruction to change a property of a parameter group to which the one or more protocols belong.

11. The method of claim 10, wherein the displayed protocols have a tree structure.

12. The method of claim 10, wherein a first protocol among the protocols is grouped in a first column of the displayed protocols, and

a second protocol among the protocols is grouped in a second column of the displayed protocols, the second protocol referring to the first protocol.

13. The method of claim 10, further comprising displaying values of the input parameter in a first column of the displayed protocols,
wherein each of the protocols is grouped to correspond to a respective one among the values, in a second column of the displayed protocols.

**Patentansprüche**

1. Medizinische Bildgebungsvorrichtung, die Folgendes umfasst:

   eine Anzeige (112), die zum Anzeigen einer Liste von Protokollen (310) ausgelegt ist, wobei jedes der Protokolle einen oder mehrere Parameter zum Erfassen eines medizinischen Bildes aufweist;
   eine Schnittstelle (111), die zum Empfangen einer Eingabe eines Parameters zum Erfassen des medizinischen Bildes ausgelegt ist; und
   eine Steuerung (121), die zum Gruppieren der Protokolle basierend auf dem eingegebenen Parameter und zum Steuern der Anzeige, um die Liste der gruppierten Protokolle anzuzeigen, ausgelegt ist,
   wobei die Schnittstelle (111) weiterhin dazu ausgelegt ist, eine Anweisung zum Ändern eines Wertes eines Parameters eines oder mehrerer der Protokolle unter den Protokollen zu empfangen, und die Steuerung (121) weiterhin dazu ausgelegt ist, den Wert des Parameters des einen bzw. der mehreren Protokolle basierend auf der Anweisung zu ändern;
   **dadurch gekennzeichnet, dass** die Anweisung zum Ändern eines Wertes eines Parameters des einen bzw. der mehreren Protokolle Folgendes umfasst: eine Anweisung zum Bewegen des einen bzw. der mehreren Protokolle aus einer Parametergruppe, zu der das eine bzw. die mehreren Protokolle gehören, in eine andere Parametergruppe, und/oder eine Anweisung zum Ändern einer Eigenschaft einer Parametergruppe, zu der das eine bzw. die mehreren Protokolle gehören.

2. Medizinische Bildgebungsvorrichtung nach Anspruch 1, wobei die angezeigte Liste der gruppierten Protokolle eine Baumstruktur aufweist.

3. Medizinische Bildgebungsvorrichtung nach Anspruch 1, wobei die Anzeige weiterhin dazu ausgelegt ist, in der angezeigten Liste der Protokolle den einen bzw. die mehreren Parameter jedes der Protokolle gemeinsam mit den jeweiligen Protokollen anzuzeigen.

4. Medizinische Bildgebungsvorrichtung nach Anspruch 1, wobei der eine bzw. die mehreren Parameter jedes der Protokolle einen Kontrast, eine Auflösung, eine Geometrie, eine Art der Sequenz, eine Schnittbildinformation, eine Fotografierichtung und/oder ein Objekt, auf das Bezug genommen wird, umfasst, und
der eingegebene Parameter den Kontrast, die Auflösung, die Geometrie, die Art der Sequenz, die Schnittbildinformation, die Fotografierichtung und/oder das Objekt, auf das Bezug genommen wird, umfasst.

5. Medizinische Bildgebungsvorrichtung nach Anspruch 1, wobei ein erstes Protokoll unter den Protokollen in eine erste Spalte der angezeigten Liste der gruppierten Protokolle gruppiert wird, und
ein zweites Protokoll unter den Protokollen in eine zweite Spalte der angezeigten Liste der gruppierten Protokolle gruppiert wird, wobei das zweite Protokoll auf das erste Protokoll Bezug nimmt.

6. Medizinische Bildgebungsvorrichtung nach Anspruch 1, wobei die Schnittstelle weiterhin konfiguriert ist zum Empfangen einer Auswahl eines Protokolls aus den Protokollen, und
die Steuerung weiterhin dazu ausgelegt ist, basierend auf dem ausgewählten Protokoll eine Impulssequenz zu erzeugen und eine Bildverarbeitung durchzuführen.

7. Medizinische Bildgebungsvorrichtung nach Anspruch 1, wobei die Steuerung weiterhin dazu ausgelegt ist, die Anzeige zu steuern, um Werte des eingegebenen Parameters in einer ersten Spalte der angezeigten Liste der gruppierten Protokolle anzuzeigen, und
jedes der Protokolle derart gruppiert wird, dass es einem jeweiligen Wert aus den Werten in einer zweiten Spalte der angezeigten Liste der gruppierten Protokolle entspricht.

8. Medizinische Bildgebungsvorrichtung nach Anspruch 1, wobei die Schnittstelle weiterhin dazu ausgelegt ist, eine Anweisung zum Ändern eines Wertes aus den Werten zu empfangen, und
die Steuerung weiterhin dazu ausgelegt ist, basierend auf der Anweisung den Wert für die gruppierten Protokolle derart zu ändern, dass er diesem Wert entspricht.

9. Medizinische Bildgebungsvorrichtung nach Anspruch 1, wobei die Anzeige weiterhin dazu ausgelegt ist, in der angezeigten Liste der gruppierten Protokollen den einen bzw. die mehreren Parameter jedes der Protokolle gemeinsam mit den jeweiligen gruppierten Protokollen anzuzeigen.

10. Verfahren zur Steuerung einer medizinischen Bildgebungsvorrichtung, wobei das Verfahren Folgendes umfasst:

Empfangen einer Eingabe eines Parameters zur Erfassung eines medizinischen Bildes;
Gruppieren von Protokollen basierend auf dem eingegebenen Parameter, wobei jedes der Protokolle einen oder mehrere Parameter zum Erfassen des medizinischen Bildes aufweist; und
Anzeigen der gruppierten Protokolle,
wobei das Verfahren weiterhin Folgendes umfasst: Empfangen einer Anweisung zum Ändern eines Wertes eines Parameters eines oder mehrer Protokolle unter den Protokollen, und zum Ändern des Wertes des Parameters des einen bzw. der mehreren Protokolle basierend auf der Anweisung;
**dadurch gekennzeichnet, dass** die Anweisung zum Ändern eines Wertes eines Parameters des einen bzw. der mehreren Protokolle Folgendes umfasst: eine Anweisung zum Bewegen des einen bzw. der mehreren Protokolle aus einer Parametergruppe, zu der das eine bzw. die mehreren Protokolle gehören, in eine andere Parametergruppe, und/oder eine Anweisung zum Ändern einer Eigenschaft einer Parametergruppe, zu der das eine bzw. die mehreren Protokolle gehören.

11. Verfahren nach Anspruch 10, wobei die angezeigten Protokolle eine Baumstruktur aufweisen.

12. Verfahren nach Anspruch 10, wobei ein erstes Protokoll aus den Protokollen in eine erste Spalte der angezeigten Protokolle gruppiert wird, und
ein zweites Protokoll aus den Protokollen in eine zweite Spalte der angezeigten Protokolle gruppiert wird, wobei das zweite Protokoll auf das erste Protokoll Bezug nimmt.

13. Verfahren nach Anspruch 10, das weiterhin Folgendes umfasst: Anzeigen von Werten des eingegebenen Parameters in einer ersten Spalte der angezeigten Protokolle,
wobei jedes der Protokolle derart gruppiert wird, dass es einem jeweiligen Wert aus den Werten in einer zweiten Spalte der angezeigten Protokolle entspricht.

**Revendications**

1. Dispositif d'imagerie médicale comprenant :

un écran (112) conçu pour afficher une liste de protocoles (310), chacun des protocoles disposant d'un ou plusieurs paramètres permettant d'acquérir une image médicale,

une interface (111) conçue pour recevoir l'entrée d'un paramètre permettant d'acquérir l'image médicale, et

un organe de commande (121) conçu pour regrouper les protocoles compte tenu de l'entrée de paramètre, et pour commander à l'écran d'afficher la liste de protocoles regroupés ;

ladite interface (111) étant conçue en outre pour recevoir une instruction visant à changer une valeur d'un paramètre d'un ou plusieurs protocoles parmi lesdits protocoles, et l'organe de commande (121) est conçu en outre pour changer la valeur du paramètre des un ou plusieurs protocoles compte tenu de l'instruction ;

**caractérisé en ce que** l'instruction visant à changer une valeur d'un paramètre des un ou plusieurs protocoles comprend une instruction visant à faire passer les un ou plusieurs protocoles, d'un groupe paramétrique auquel les un ou plusieurs protocoles appartiennent vers un autre groupe paramétrique, et/ou une instruction visant à changer une propriété d'un groupe paramétrique auquel les un ou plusieurs protocoles appartiennent.

2.  Dispositif d'imagerie médicale selon la revendication 1, dans lequel la liste affichée de protocoles regroupés a une structure arborescente.

3.  Dispositif d'imagerie médicale selon la revendication 1, dans lequel l'écran est conçu en outre pour afficher, dans la liste affichée de protocoles, les un ou plusieurs paramètres de chacun des protocoles conjointement aux protocoles respectifs.

4.  Dispositif d'imagerie médicale selon la revendication 1, dans lequel les un ou plusieurs paramètres de chacun des protocoles concernent au moins un des éléments suivants : le contraste, la résolution, la géométrie, le type de séquence, des informations de vue anatomique, le sens de prise de vue et l'objet auquel il est fait référence, et

l'entrée de paramètre concerne au moins un élément parmi : le contraste, la résolution, la géométrie, le type de séquence, des informations de vue anatomique, le sens de prise de vue et l'objet auquel il est fait référence.

5.  Dispositif d'imagerie médicale selon la revendication 1, dans lequel un premier protocole au sein des protocoles est regroupé dans une première colonne de la liste affichée de protocoles regroupés, et

un deuxième protocole au sein des protocoles est regroupé dans une deuxième colonne de la liste affichée de protocoles regroupés, le deuxième protocole faisant référence au premier protocole.

6.  Dispositif d'imagerie médicale selon la revendication 1, dans lequel l'interface est conçue en outre pour recevoir la sélection d'un protocole parmi les protocoles, et

l'organe de commande est conçu en outre pour produire une séquence d'impulsions et réaliser un traitement d'image, compte tenu du protocole sélectionné.

7.  Dispositif d'imagerie médicale selon la revendication 1, dans lequel l'organe de commande est conçu en outre pour commander à l'écran d'afficher des valeurs de l'entrée de paramètre dans une première colonne de la liste affichée de protocoles regroupés, et

chacun des protocoles est regroupé pour correspondre à une valeur respective parmi les valeurs, dans une deuxième colonne de la liste affichée de protocoles regroupés.

8.  Dispositif d'imagerie médicale selon la revendication 1, dans lequel l'interface est conçue en outre pour recevoir une instruction visant à changer une valeur parmi les valeurs, et

l'organe de commande est conçu en outre pour changer la valeur relative aux protocoles regroupés afin qu'elle corresponde à la valeur, compte tenu de l'instruction.

9.  Dispositif d'imagerie médicale selon la revendication 1, dans lequel l'écran est conçu en outre pour afficher, dans la liste affichée de protocoles regroupés, les un ou plusieurs paramètres de chacun des protocoles conjointement aux protocoles regroupés respectifs.

10. Procédé de commande d'un dispositif d'imagerie médicale, le procédé comprenant :

la réception de l'entrée d'un paramètre permettant d'acquérir une image médicale,

le regroupement des protocoles compte tenu de l'entrée de paramètre, chacun des protocoles disposant d'un ou plusieurs paramètres permettant d'acquérir l'image médicale, et

l'affichage des protocoles regroupés,

le procédé comprenant en outre la réception d'une instruction visant à changer une valeur d'un paramètre d'un ou plusieurs protocoles parmi lesdits protocoles, et le changement de la valeur du paramètre des un ou plusieurs protocoles compte tenu de l'instruction ;

**caractérisé en ce que** l'instruction visant à changer une valeur d'un paramètre des un ou plusieurs protocoles comprend une instruction visant à faire passer les un ou plusieurs protocoles, d'un groupe paramétrique auquel les un ou plusieurs protocoles appartiennent vers un autre groupe paramétrique, et/ou une instruction visant à changer une propriété d'un groupe paramétrique auquel les un ou plusieurs protocoles appartiennent.

11. Procédé selon la revendication 10, dans lequel les protocoles affichés ont une structure arborescente.

12. Procédé selon la revendication 10, dans lequel un premier protocole au sein des protocoles est regroupé dans une première colonne de protocoles affichés, et
un deuxième protocole au sein des protocoles est regroupé dans une deuxième colonne de protocoles affichés, le deuxième protocole faisant référence au premier protocole.

13. Procédé selon la revendication 10, comprenant en outre l'affichage de valeurs de l'entrée de paramètre dans une première colonne de protocoles affichés, chacun des protocoles étant regroupé pour correspondre à une valeur respective parmi les valeurs, dans une deuxième colonne de protocoles affichés.

**FIG. 1**

# FIG. 2

# FIG. 3

# FIG. 4

# FIG. 5

**FIG. 6**

# FIG. 7

310

| Protocol List | C1 | C2 | C3 |
|---|---|---|---|
| Protocol A | | | − |
| Protocol B | | | A |
| Protocol C | | | A |
| Protocol D | | | C |
| Protocol E | | | C |
| Protocol F | | | A |
| Protocol G | | | F |
| Protocol H | | | G |
| Protocol I | | | A |
| Protocol J | | | I |

# FIG. 8

# FIG. 9

310

C2

Protocol C

Protocol I

Protocol A

Protocol B

Protocol D

Protocol F

Protocol G

Protocol J

Protocol E

Protocol H

# FIG. 10

# FIG. 11

310

# FIG. 12

# FIG. 13

310

C1

Protocol A

Protocol C

Protocol F

Protocol I

Protocol E

Protocol J

Protocol B

Protocol D

Protocol G

Protocol H

Non-grouping Protocols

Protocol K

# FIG. 14

310

# FIG. 15

# FIG. 16

310a

| | |
|---|---|
| ▨ | Protocol A<br>Protocol C<br>Protocol F<br>Protocol I |
| ▤ | Protocol E<br>Protocol J |
| ▥ | Protocol B<br>Protocol D<br>Protocol G<br>Protocol H |

# FIG. 17

310b

| |
|---|
| T1_Protocol A |
| T1_Protocol C |
| T1_Protocol F |
| T1_Protocol I |
| T2_Protocol E |
| T2_Protocol J |
| PD_Protocol B |
| PD_Protocol D |
| PD_Protocol G |
| PD_Protocol H |

# FIG. 18

310c

| | | | |
|---|---|---|---|
| Protocol A | | | − |
| Protocol C | | | A |
| Protocol F | | | A |
| Protocol I | | | A |
| Protocol E | | | C |
| Protocol J | | | I |
| Protocol B | | | A |
| Protocol D | | | C |
| Protocol G | | | F |
| Protocol H | | | G |

**FIG. 19**

# FIG. 20

# FIG. 21

```
                    ( START )
                        |
                        v
    +-------------------------------------+
    |         REGISTER PATIENT            |--S1100
    +-------------------------------------+
                        |
                        v
    +-------------------------------------+
    |   RECEIVE SELECTION OF CRITERION    |--S1200
    |      FOR CLASSIFYING PROTOCOLS      |
    +-------------------------------------+
                        |
                        v
    +-------------------------------------+
    |       RECEIVE INSTRUCTION TO        |--S1300
    |   CHANGE PARAMETER OF PROTOCOL      |
    +-------------------------------------+
                        |
                        v
    +-------------------------------------+
    |     RECEIVE SELECTION OF PROTOCOL   |--S1400
    +-------------------------------------+
                        |
                        v
    +-------------------------------------+
    |  ACQUIRE MAGNETIC RESONANCE IMAGE   |--S1500
    +-------------------------------------+
                        |
                        v
                    (  END  )
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2713177 A